# EUROPEAN PATENT APPLICATION

(11) **EP 1 224 933 A1**
(43) Date of publication of application: **24.07.2002**
(21) Application number: 01400166.3
(22) Date of filing: 19.01.2001
(51) Int. Cl.: A61K 31/505, A61P 15/10

(54) **New combination of active ingredients containing alfuzosine and apomorphine**

(71) Applicant: SANOFI-SYNTHELABO, 75013 Paris (FR)
(72) Inventor: Arbilla, Sonia, 75007 Paris (FR); Ramirez, Juan-Fernando, Sanofi-Synthelabo, 75013 Paris (FR)
(74) Representative: Ludwig, Jacques

(57) **Abstract**

The subject of the present invention is a new combination of active ingredients consisting of alfuzosine and apomorphine, and pharmaceutical compositions containing them, for ameliorating and/or treating erectile dysfunction in male patients.

## Description

The subject of the present invention is a new combination of active ingredients consisting of alfuzosine and apomorphine, and pharmaceutical compositions containing them, for ameliorating and/or treating erectile dysfunction in male patients.

The active ingredients constituting the combination are present in the free state or in the form of one their salts.

Alfuzosine and its salts, particularly the hydrochloride are known drugs used in the symptomatic treatment of benign hypertrophy of the prostate.

Apomorphine is a selective dopamine receptor agonist that has been widely utilized as an emetic agent, sedative, antiparkinsonian agent and a behavior altering agent. Recent research and clinical studies have demonstrated that in males apomorphine has an erectogenic effect manifested by penile erection. However these studies show that while apomorphine can indeed induce an erection in a psychogenic male patient, the apomorphine dose required to achieve a significant erectile response is usually accompanied by nausea or other serious undesirable side effects such as hypertension, flushing and diaphoresis.

Quite surprisingly and unexpectedly, the alfuzosine-apomorphine combination of the invention proved to be endowed with a synergistic activity of the two active ingredients in ameliorating and/or treating male erectile dysfunction. Therefore the adverse side-effects, seen with apomorphine, of nausea, hypotension and sedation is minimised by use of as low a dose as possible.

The combinations according to the invention can be formulated in pharmaceutical compositions for administration to mammals, including man, for the ameliorating and/or treating the above mentioned dysfunction, which is another object of the present invention.

According to the invention, alfuzosine and apomorphine can be administered in the form of a salt.

These salts include for example salts with mineral acids such as hydrochloric acid, hydrobromic acid, sulfuric acid, nitric acid, phosphoric acid; salts with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, cinnamic acid, lactic acid, glycolic acid, glucuronic acid, ascorbic acid, nicotinic acid, and salicylic acid; or salts with acidic amino acids such as aspartic, and glutamic acid. Pharmacological acceptable salts an preferred.

In the text which follows, the quantities of alfuzosine and of apomorphine are expressed as alfuzosine and apomorphine equivalents in non-salified, free form.

Advantageously, the composition of the invention comprise alfuzosine and apomorphine in a molar ratio (alfuzosine/apomorphine) of 0.1 to 20, preferably of 0.2 to 10.

In human beings, the dose may vary for each of the active ingredient from 0.5 to 25 mg per day, depending on the age of the subject to be treated.

In the pharmaceutical compositions of the present invention, the active ingredients are generally formulated in dosage units containing from 0.1 to 25 mg of the said active ingredient per unit dosage.

The subject of the present invention is therefore the pharmaceutical compositions which contain, as active ingredients, a combination of alfuzosine and apomorphine. These compositions are preferably made so as to be administerable by the oral or parenteral route.

In the pharmaceutical compositions of the present invention for oral, sublingual, subcutaneous, intramuscular, intravenous, intradermal, local or rectal administration, the active ingredient may be administered in unit forms for administration, mixed with conventional pharmaceutical carriers, to animals and to human beings. For example, the pharmaceutical composition may be formulated, for example, in the form of pharmaceutical compositions for oral administration such as granules, fine granules, powders, hard capsules, soft capsules, syrups, emulsions, suspensions, solutions and the like, or in the form for sublingual a trucal administration, or in the form of pharmaceutical compositions for parenteral administrations such as injections for intravenous, intramuscular, or subcutaneous administration, drip infusions, transdermal preparations, transmucosal preparations, nasal drops, inhalants, suppositories and the like. Injections or drip infusions may be prepared as powdery preparations such as in the form of lyophilized preparations, and may be used by dissolving just before use in an appropriate aqueous medium such as physiological saline. Sustained-release preparations such as those coated with a polymer may be directly administered intracerebrally.

Types of pharmaceutical additives used for the manufacture of the pharmaceutical composition, content ratios of the pharmaceutical additives relative to the active ingredient, and methods for preparing the pharmaceutical composition may be appropriately chosen by those skilled in the art. Inorganic or organic substances, or solid or liquid substances may be used as pharmaceutical additives. Generally, the pharmaceutical additives may be incorporated in a ratio ranging from 1% by weight to 90% by weight based on the weight of an active ingredient.

Examples of excipients used for the preparation of solid pharmaceutical compositions include, for example, lactose, sucrose, starch, talc, cellulose, dextrin, kaolin, calcium carbonate and the like. When a solid composition in the form of tablets is prepared, the active ingredients are mixed with the excipients. The tablets can be coated with sucrose or other appropriate materials or alternatively they can be treated such that they have a prolonged or delayed activity and that they have a prolonged or delayed activity and that they continuously liberate a predetermined quantity of active ingredient.

A preparation in the form of gelatin capsules is obtained by mixing the active ingredient with a diluent and by pouring the mixture obtained into soft or hard gelatin capsules. For the preparation of liquid compositions for oral administration, a conventional inert diluent such as water or a vegetable oil may be used. The liquid composition may contain, in addition to the inert diluent, auxiliaries such as moistening agents, suspension aids, sweeteners, aromatics, colorants, and preservatives. The liquid composition may be filled in capsules made of an absorbable material such as gelatin. Examples of solvents or suspension mediums used for the preparation of compositions for parenteral administration, e.g. injections, suppositories, include water, propylene glycol, polyethylene glycol, benzyl alcohol, ethyl oleate, lecithin and the like. Examples of base materials used for suppositories include, for example, cacao butter, emulsified cacao butter, lauric lipid, witepsol.

The dose and frequency of administration of the medicament of the present invention are not particularly limited, and they may be appropriately chosen depending on conditions such as the body weight or age of a patient, severity and the like. Generally, a daily dose for oral administration may be administered once a day or several times a day as divided portions, or once in several days.

When the composition of the invention are administered in man by the parenteral or oral route, it is preferable that the daily dose of alfuzosine is between 1 and 15 mg, the daily dose of apomorphine being between 1 and 4 mg.

It will be noted that according to the invention, alfuzosine and apomorphine can both be administered by the oral route, or both by the parenteral route or one can be administered by the oral route (preferably alfuzosine) and the other by the parenteral route (preferably apomorphine).

According to a preferred embodiment, the daily dose of alfuzosine administered in man by the parenteral and/or oral route is between 1 and 15 mg, better still between 2.5 and 10 mg, the daily dose of apomorphine administered by the parenteral route being between 1 and 4 mg, better still between 1.5 and 3 mg.

According to another of its aspects, the present invention also relates to a method for treating/ameliorating the pathologies indicated above, which comprises the administration to a patient of an effective amount of the combination according to the invention.

The combinations of active ingredients according to the invention have been the subject of pharmacological studies.

### Examples

These studes were carried out in order to determine whether the erectile responses induced by apomorphine could be potentiated by alfuzosine administration in hypertensive rats (SHR).

### Material and methods

### Animals and experimental protocol

Male SHR, 17 weeks old, from Charles River (France) weighing 310 to 400 g were used. Before the experiment, the animals were housed as groups of 4 for at least one week. Dry pellets and tap water were available ad libitum. The day of experiment, animals were anaesthetised with 6% sodium pentobarbital at a dose of 20 mg/kg i.p. completed with ketamine 1000 at a dose of 20 mg/kg i.p. To prevent any pain during surgery, subcutaneous lidocaine 2.13% injections were done as needed (1 ml maximum). Each rat was placed on a heated blanket at 38°C (Harvard, model 50-80210).

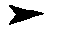 ***Surgical procedure***

After insensibilization with lidocaine, inguinal skin was incised and a polyethylene catheter (Biotrol nb4) was implanted in the femoral artery for mean blood pressure recording. The catheter was filled with heparine 500 Ul/ml in 0.9% NaCl. After insensibilization with lidocaine, scrotal skin was incised between the testis and a needle (26G) connected to a catheter (Biotrol nb1) was implanted in the corpus cavernosum through a purse-string suture on the tunica albuginea for intracavernous pressure (ICP) recording. The catheter was filled with the heparinized saline.

Intraarterial and intracavernous catheters were connected via Spectramed Statham (P23XL) or Medex (MX860) pressure transducers to a Grass polygraph (model 7). The signals acquired by the Grass polygraph were digitised by a MacLab/8e and analysed by «Chart 3.5.7» running on Power Macintosh 8500/180 or G3 266MHz.

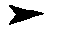 ***Administration of treatments***

Apomorphine was prepared in saline with 0.2% ascorbic acid and administered subcutaneously at 0.5 ml/kg. Alfuzosine was prepared in 5% glucose and i.v. injection was made as 5 min infusion at 0.2ml/min.

After a 20 min steady-state following the surgery, apomorphine or its vehicle was administered subcutaneously; followed 20 min later, by the infusion of alfuzosine or its vehicle intravenously during 5 min. The effect of the infusion was monitored for 30 min.

### Variables measured

Mean blood pressure and intracavernous pressure were continuously monitored. Mean blood pressure was recorded during 30 seconds every 5 minutes after the administration of compounds.
To describe the erectile responses, the following parameter were analysed: the number of erections and for each erection the AUC (air under the curve), the duration and the amplitude expressed as delta ICP (*maximal intracavernous pressure - basal intracavernous pressure*).

### Compounds

Apomorphine
Alfuzosine
6% Pentobarbital: Sanofi-Santé animale,
Ketamin : Imalgène®1000 Rhône Poulenc
Lidocaïne : Xylovet® Sanofi
Ascorbic Acid: PROLABO
5% glucose: Fresenius France Pharma (Biosedra)
0.9% NaCI: Fresenius France Pharma (Biosedra)
Heparin 25000 UI (Choay)

### Statistics

Results are shown as mean ± s.e.m. Statistics were performed with SAS release 6.09 Everstat software according to preclinical biostatistic unit recommendations on raw data or using square root transformation, followed by Two way ANOVA or Two way ANOVA with repeated measures (Winer analysis). A 2 way ANOVA was performed on the rank of the mean parameters when variances were not homogeneous. A probability level < 0.05 was accepted as significant. The number of erections after infusion was a discrete variable which generally followed a Poissons distribution. To normalise this variable a square root transformation was used (Bonferroni's correction was used for statistical probability). Results on preliminary data are internally available (Guenneau, statistical study report number B00002).

### Results

Erections are characterised by their number, AUC, ICP delta and duration. The response of each animal after infusion of alfuzosine or its vehicle was very variable in terms of both number and characteristics of erections. To take into account this great variability, the parameters of all the erectile events observed for each animal after infusion of alfuzosine or its vehicle were averaged (whatever the number of erections) and used for statistical comparison.

### Effect of apomorphine

Apomorphine was administered at 10, 20, 30, 50, 100, or 250µg/kg s.c., followed by a 5 min infusion of 5% glucose, the control group receiving vehicule (ascorbic acid 0.2%) plus 5% glucose. Each rat received only one treatment.

### Number of erections

In some animals, apomorphine triggered erections as early as within 20 min after subcutaneous administration. In order to be able to evaluate the effect of alfuzosine, these early events were not considered and erections were monitored only after the infusion of 5% glucose and then recorded during 30 min.
In the control group (ascorbic acid / glucose), there were no erections, the variance was null. Apomorphine 30µg/kg was the first significant dose increasing the number of erections in comparison to the control group. Apomorphine 50µg/kg was the first dose which induced at least one erectile event in all the animals of this group (Table 1).

**Table 1 -**

| Effect of apomorphine on erection parameters | | | | | | |
|---|---|---|---|---|---|---|
| Treatment | Number of animals | N animals with erections/ N animals | Number of erections (mean ± sem) | AUC (cmH₂O.sec) | ΔICP (cmH₂O) | Duration (sec) |
| Asc. Ac./5% Glc | 6 | 0/6 | 0 | 0 | 0 | 0 |
| Apo 10 / " | 8 | 0/8 | 0 | 0 | 0 | 0 |
| " 20 / " | 10 | 5/10 | 0.5000 ± 0.1667 | 582 ± 172 | 41.4 ± 13.9 | 61 ± 7* |
| " 30 / " | 11 | 7/11 | 1.0000 ± 0.3015* | 908 ± 399 | 64.0 ± 11.3* | 33 ± 5* |
| " 50 / " | 9 | 9/9 | 2.5556 ± 0.3768* | 640 ± 215 | 29.3 ± 6.5* | 105 ± 23* |
| " 100/ " | 9 | 9/9 | 3.1111 ± 0.3514* | 501 ± 193 | 25.5 ± 8.2 | 71 ± 19* |
| " 250/ " | 6 | 6/6 | 5.0000 ± 0.5164* | 1073 ± 237* | 58.6 ±9.7* | 71 ± 19 |

| | | | | | | |
|---|---|---|---|---|---|---|
| Asc = ascorbic acid; Glc = glucose; Apo = apomorphine | | | | | | |
| *p<0.05 (Bonferroni's method) | | | | | | |

On the parameter « number of erections », the ED₅₀ for apomorphine was calculated to be 36 µg/kg s.c..

### Mean blood pressure

Mean blood pressure was recorded 30 seconds before the administration of apomorphine (-20 min) and before the administration of vehicle (-5 min), and every 5 minutes after the perfusion of vehicle.

A two way ANOVA was performed for each dose of apomorphine on the time with -20min as control.

Administration of vehicle did not modify mean blood pressure throughout the experiment. At 10 and 20 µg/kg s.c., apomorphine did not modify significantly mean blood pressure, while a significant decrease in blood pressure was observed after 50 µg/kg (p<0.05 lasting for 15 min) and 100 µg/kg (p<0.05 maintained up to the end of experiment).

### Effect of alfuzosine

The vehicle of apomorphine (ascorbic acid 0.2%) was administered s.c., followed 20 min after by a 5 min infusion of alfuzosine at 3, 10, 30 or 100 µg/kg i.v., the control group receiving vehicle (ascorbic acid 0.2% + 5% glucose).

### Number of erections

For each animal the number of erections was counted after 5% glucose or alfuzosine infusion. Neither 5% glucose nor alfuzosine (3 to 100 µg/kg i.v.) was able to induce per se erections.

### Mean blood pressure

Mean blood pressure was recorded for 30 seconds before the administration of alfuzosine i.v. (-5 min) and every 5 minutes during 30 min after the perfusion of the compound.
A one way ANOVA was performed on the time -5minutes to verify the homogeneity of the different groups before the administration of alfuzosine. To study the effect of different doses of alfuzosine on the arterial pressure during the 30 minutes after the infusion a two way ANOVA with repeated measures on time compared to the control group was realised for each dose. Basal values of arterial pressure before infusion were not different (F(4,25)=0.07 , p=0.99).
Alfuzosine 3µg/kg iv had no effect on arterial pressure except at the time +30 minutes
(- 34%, p=0.0251).
Alfuzosine 10µg/kg iv decreased significantly the arterial pressure from the end of infusion (-23% , p=0.0458) to the end of experiment (-29%, p=0.0186).
Alfuzosine 30µg/kg iv decreased significantly the arterial pressure from 5 minutes after the end of infusion (-28%, p=0.0460) to the end of experiment (-34%, p=0.0254).
Alfuzosine 100µg/kg iv decreased significantly the arterial pressure from 5minutes to 20 minutes after the end of infusion.

### Potentiation of the effect of apomorphine by alfuzosine

### Effects of alfuzosin after apomorphine 50µg/kg

Apomorphine was administered at 50µg/kg s.c., followed by a 5 min infusion of 5% glucose (control group) or alfuzosine (3, 10 or 30µg/kg). This dose of apomorphine was tested because it was the first dose which provoked at least one erection after infusion in all animals. Each rat received only one treatment. The characteristics of erections in control group are given in Table 1

### Number of erections

For each animal the number of erections was counted after infusion.
In spite of the lack of statistical significance, the number of erections tended to increase progressively with the dose of alfuzosine (Table 2).

### Erectile parameters

In spite of the lack of significant effects on the number of erections, the quality of erections (AUC, ICP delta and duration) after the association of apomorphine s.c. and alfuzosine i.v. was studied.

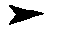 *Effect on AUC*

A Dunnett's test was realised to compare each apomorphine-alfuzosine association with apomorphine alone.
At 10 and 30µg/kg i.v., alfuzosine significantly potentiated the effect of 50µg/kg s.c. apomorphine on erectile parameters expressed as AUC (p<0.05) (Table 2).

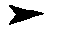 *Effect on delta ICP*

Alfuzosine associated with apomorphine 50µg/kg increased significantly the delta ICP compared to apomorphine alone. This effect of alfuzosine, although not dose dependent, was statistically significant at 3 and 30 µg/kg (p<0.05) (Table 2).

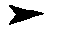 *Effect on duration*

Alfuzosine (3-30 µg/kg i.v.) associated with apomorphine 50µg/kg did not change the mean duration of erections compared to apomorphine alone (Table 2).

**Table 2 :**

| Effects of apomorphine, alfuzosin and their association on the number of erections, intra-cavernous pressure (ICP) and erection duration. | | | | | | |
|---|---|---|---|---|---|---|
| Treatment | n | n animal with erections / n animal | number of erections | Δ ICP (cmH₂O) | Duration of erection (sec) | AUC (cmH₂O.sec) |
| Control solvant | 6 | 0/6 | 0 | 0 | 0 | 0 |
| Apomorphine (50µg/kg) | 9 | 9/9 | 2.6±0.4 | 29±7 | 105±23 | 640±216 |
| Alfuzosine (3, 10 or 30 µg/kg) | 6 | 0/6 | 0 | 0 | 0 | 0 |
| Apomorphine (50µg/kg)+ Alfuzosine (3 µg/kg) | 6 | 6/6 | 3.3±0.7 | 54±11* | 125±22 | 397±184 |
| Apomorphine (50µg/kg)+ Alfuzosine (10 µg/kg) | 6 | 5/6 | 3.2±0.7 | 52±17 | 181±22 | 1760±508* |
| Apomorphine (50µg/kg) + Alfuzosine (30 µg/kg) | 10 | 10/10 | 4±0.4⁽¹⁾ | 55±5* | 103±14 | 1406±284* |
| * Statistically different from Apomorphine (50µg/kg), p<0.05 ⁽¹⁾ p = 0.0531 ; | | | | | | |

### Mean blood pressure

There were no significant differences between the experimental groups in mean blood pressure measured before the infusion of either glucose or increasing doses of alfuzosine. Administration of alfuzosine did not modify the mean blood pressure after apomorphine 50µg/kg s.c. except at the time +15 minutes for the 3µg/kg dose of alfuzosine (p=0.0480) (data not shown).

### Conclusion

Anaesthetised SHR did not show spontaneous erections. In this experimental model, apomorphine (10-250 µg/kg s.c.) induced concentration-dependent erectile responses. Apomorphine 30µg/kg s.c. was the first dose which increased significantly the number of erections in this model while at 50µg/kg all the groups showed at least one erection.

Alfuzosine (10 or 30µg/kg i.v.) in association with apomorphine 50µg/kg s.c. increased significantly the mean AUC when compared to apomorphine alone. This increase of mean AUC observed with alfuzosine 30µg/kg was associated with an increase of delta ICP. Apomorphine (50µg/kg s.c.) alone induced antihypertensive effects, however the administration of alfuzosine (3-30µg/kg i.v.). did not modify further the mean blood pressure in comparison to apomorphine alone.

In conclusion, in anesthetised SHR, under conditions in which alfuzosine is unable to induce erectile responses on its own, alfuzosine has been demonstrated to potentiate the erectile response induced by submaximal doses of apomorphine.

## Claims

1. Pharmaceutical composition containing a combination of active ingredients in which the active ingredients are alfuzosine and apomorphine and both active ingredients being present in the free state or in the form of a salt.

2. Pharmaceutical composition according to Claim 1, in a form administerable by the parenteral route or by the oral route.

3. Pharmaceutical composition according either claim 1 or 2 **characterized in that** alfuzosine and apomorphine are of molar ratio of 0.2 to 10.

4. Pharmaceutical composition according to any one of the preceding claims, for the treatment or amelioration of erectile dysfunction.

5. Use of a composition according to any one of the preceding claims, for the preparation of a medicament intended for the treatment or amelioration of erectile dysfunction.

6. Use according to claim 5, in which the treatment involves the administration by the parenteral and/or oral route of 1 to 15 mg of alfuzosine per day and of 1 to 4 mg of apomorphine per day.
